# EUROPÄISCHE PATENTSCHRIFT

(11) **EP 0 647 699 B1**
(45) Veröffentlichungstag und Bekanntmachung des Hinweises auf die Patenterteilung: **23.12.1998**
(21) Anmeldenummer: 94250241.0
(22) Anmeldetag: 07.10.1994
(51) Int. Cl.: C10G 9/16

(54) **Verfahren zur Verminderung der Verkokung von Wärmetauschflächen**
Process for reducing coking of heat transfer surfaces
Procédé pour diminuer la cokéfaction de surfaces d'échange de chaleur

(30) Priorität: 08.10.1993 DE 4334827
(43) Veröffentlichungstag der Anmeldung: 12.04.1995
(73) Patentinhaber: MANNESMANN Aktiengesellschaft, 40213 Düsseldorf (DE); KTI GROUP B.V., NL-2700 AB Zoetermeer (NL)
(72) Erfinder: Barendregt, Simon, NL-3161 VR Rhoon (NL); Zimmermann, Gerhard, D-04299 Leipzig (DE); Bach, Grete, D-04157 Leipzig (DE)
(74) Vertreter: Presting, Hans-Joachim, Dipl.-Ing.

(56) Entgegenhaltungen:
- EP-A- 0 022 349
- EP-A- 0 110 486

## Beschreibung

Die Erfindung betrifft ein Verfahren zur Verminderung der Verkokung von Wärmetauschflächen in einem Röhrenwärmetauscher gemäß dem Oberbegriff des Patentanspruchs 1.

Beispielsweise zur Herstellung des Chemierohstoffs Äthylen wird ein thermisches Cracken von höheren Kohlenwasserstoffen in entsprechenden Cracköfen vorgenommen. Die erhaltenen heißen Crackprodukte, die in einem solchen Fall zu einem großen Teil aus Äthylen bestehen, werden nach Verlassen des Crackofens abgekühlt. Hierzu werden Wärmetauscher für einen indirekten Wärmetausch eingesetzt, die zur Erzeugung von nutzbarem Dampf mit Wasser als Kühlmittel betrieben werden. Derartige als Röhrenwärmetauscher aus Kesselbaustahl ausgebildete Apparate werden auch in anderen chemischen Anlagen zur Kühlung oder Beheizung von Kohlenwasserstoffprodukten benutzt.

Die Wirksamkeit des Wärmetauschers hängt stark davon ab, ob sich im Betrieb Ablagerungen auf den Stahlrohroberflächen (z.B. Rohr aus 15Mo3) des Wärmetauschers bilden, die den Wärmeübergang behindern. Dies ist bei den heute verwendeten Apparaten regelmäßig der Fall. Nach bestimmten Betriebszeiten zeigt sich auf der mit den Kohlenwasserstoffen in Kontakt tretenden Seite der Wärmetauschflächen eine zunehmende Verkokung. Daher wird es immer wieder notwendig, bei Erreichen eines bestimmten Grades an Leistungsverminderung den betreffenden Wärmetauscher außer Betrieb zu nehmen und einer aufwendigen Reinigungsprozedur zu unterziehen. In modernen Anlagen wird dies häufig in der Weise durchgeführt, daß ein Gemisch aus heißem Wasserdampf und Luft auf der verkokten Seite durch den Wärmetauscher geführt wird, wodurch die gebildeten Ablagerungen abgelöst und ausgetragen werden, so daß wieder eine metallische Oberfläche zur Verfügung steht, die einen guten Wärmeübergang von den heißen Crackprodukten in die Rohrwände des Wärmetauschers gewährleisten.

Trotz gründlichster Reinigung können bereits kurz nach Wiederinbetriebnahme des Wärmetauschers neu gebildete Kohlenstoffablagerungen festgestellt werden, so daß nach relativ kurzen Betriebszeiten (z.B. nach 20 bis 60 Tagen) erneut eine Reinigung erfolgen muß. Dies ist aus technischer wie aus wirtschaftlicher Sicht gleichermaßen unerwünscht, da nicht nur eine längerfristig stationäre Betriebsweise behindert wird, sondern auch die effektive Anlagennutzung verringert wird und darüber hinaus auch die Kosten für den Reinigungsvorgang selbst sehr häufig anfallen. Aus diesem Grunde ist man seit Jahren bemüht, Lösungen für das Problem der Verhinderung einer schnellen Verkokung der Wärmetauscherflächen zu finden.

Aus der EP 0 022 349 A1 ist es bekannt, die Wärmetauscheroberfläche, die zur Verkokung neigt, mit einem Schutzfilm aus Oxiden der Metalle Ca, Mg, Al, Ga, Ti, Zr, Hf, Ta, Nb oder Cr zu bedampfen. Dies ist ein relativ aufwendiges Verfahren und bringt außerdem nur einen sehr begrenzten Erfolg mit sich.

Eine ähnliche Vorgehensweise, nämlich die Aufbringung einer Schutzschicht, wird auch in der EP 0 110 486 A1 vorgeschlagen. Neben Metalloxiden werden zu diesem Zweck bestimmte Metalle, Aluminate und Silikate vorgesehen. Auch diese Lösungen brachten zwar Verbesserungen, konnten in manchen Fällen aber immer noch nicht als zufriedenstellend angesehen werden.

Eine weitere Verbesserung, die aber auch noch nicht immer als hinreichend betrachtet werden kann, ist durch eine aus der NL 84 01 804 A1 bekannte Beschichtung auf der Basis von Silikonharz gegeben, bei der Siliziumkarbid, Borkarbid, Titankarbid, Siliziumnitrid oder Bornitrid in das Harz eingelagert wird.

Schließlich ist noch auf die versuchte Verwendung Molybdän-freier Stahllegierungen für die Wärmetauscherrohre hinzuweisen. Es bestand nämlich die Vermutung, daß das in den üblichen Wärmetauscherstählen enthaltene Molybdän eine für die Verkokung förderliche katalytische Wirkung mit sich bringe. Dies hat sich jedoch nicht als zutreffend erwiesen.

Gemeinsam ist den bisherigen Lösungsvorschlägen, daß sie praktisch nur an Neuanlagen erfolgen können, nicht aber an bereits in Betrieb befindlichen Anlagen (ausgenommen bei einem Revamping).

Der Erfindung liegt daher die Aufgabe zugrunde, eine Lösung vorzuschlagen, mit der die im Betrieb der betreffenden Wärmetauscher bisher auftretende Verkokung wesentlich vermindert werden kann.

Gelöst wird diese Aufgabe durch ein Verfahren mit den Merkmalen des Patentanspruchs 1; vorteilhafte Weiterbildungen der Erfindung sind in den Unteransprüchen 2 bis 6 angegeben.

Der Erfindung liegt die überraschende Erkenntnis zugrunde, daß eine bestimmte Form des Eisenoxid, nämlich Fe₂O₃, eine sehr starke katalytische Wirkung für die Verkokung entfaltet, während ein anderes Eisenoxid, nämlich das Fe₃O₄, eine solche katalytische Wirkung praktisch nicht besitzt. Das metallische Eisen im Verband metallischer Werkstoffe hat lediglich eine mittlere Aktivität, während feinste staubförmige Eisenpartikel, die nach der Entkokung auf der Innenoberfläche der Wärmetauscherrohre in fein verteilter Form zurückbleiben, eine sehr hohe Koksbildung verursachen, wenn sie in reduzierter Form vorliegen. Maßgeblich für die stets beobachtete schnelle Verkokung der Wärmetauscherflächen ist daher das Fe₂O₃. Unerwünschterweise bildet sich dieses Oxid aber auf den Stahlrohroberflächen bei der üblichen Reinigung mittels eines heißen Wasserdampf/Luft-Gemisches. Dies führt dazu, daß gerade die frisch gereinigten Oberflächen in der Anfangsphase nach der Wiederinbetriebnahme eines Wärmetauschers eine besonders hohe Verkokungsrate aufweisen, also sehr schnell eine gewisse Schicht an Kohlenstoff ansetzen. Danach ist die Verkokungsrate geringer, da die Kohlenstoffschicht als Schutzfilm fungiert. Eine ähnlich negative Wirkung geht von den fein verteilten Eisenclustern aus, die während der Verkokung in die Koksmatrize eingebaut werden und beim Entkoken freigelegt werden. Sie können ebenfalls eine hohe Anfangsverkokung des Wärmetauschers bewirken.

Um aber gerade diese rasche Wiederbildung einer ersten Verkokungsschicht auf der Wärmetauscherfläche zu vermeiden, sieht die Erfindung vor, nach einer Reinigung des Wärmetauschers vor der Wiederinbetriebnahme eine zusätzliche Behandlung der mit den Crackprodukten in Berührung kommenden Oberflächenteile vorzunehmen, durch die das katalytisch sehr aktive Fe₂O₃ zu dem inaktiven Fe₃O₄ reduziert wird, wobei eine Reduktion von Fe-Clustern aus der Koksmatrix zu metallischem Fe-Staub (submikrones Fe-Pulver) vermieden wird. Dies geschieht zweckmäßigerweise so, daß nach der Reinigung, die in üblicher Weise zum Beispiel mit einem heißen Wasserdampf/Luft-Gemisch vorgenommen werden kann, ein Gemisch aus heißem Wasserdampf und Wasserstoff in einer solchen Zusammensetzung durch den Wärmetauscher geschickt wird, daß auf der Innenoberfläche des Wärmetauschers, die während der Pyrolyse der Verkokung ausgesetzt ist, durch eine reduzierende Atmosphäre das Fe₂O₃ gezielt in Fe₃O₄ umgewandelt wird, aber noch keine durchgreifende Reduktion der Eisenoxide zu Fe-Staub erfolgt. Wenn diese Umwandlung ein ausreichendes Ausmaß erreicht hat, kann der Wärmetauscher wieder in Betrieb genommen werden. Da die hohe katalytische Aktivität des Fe₂O₃ an der Oberfläche nicht mehr vorhanden ist, kann eine erneute Verkokung erst mit sehr großer zeitlicher Verzögerung eintreten. Durch diese vergleichsweise einfache Zusatzbehandlung nach einer üblichen Reinigung ermöglicht die vorliegende Erfindung eine erhebliche Verlängerung der Betriebszeiten der Wärmetauscher. Wesentlich dabei ist es, daß am Wärmetauscher selbst hierzu keine baulichen Veränderungen vorgenommen werden müssen und daß das Verfahren auch für bereits bestehende Anlagen anwendbar ist. Außerdem sind keinerlei Beschichtungen, die den Wärmeübergang beeinträchtigen können, erforderlich.

Es hat sich als günstig herausgestellt, das Gemisch aus Wasserdampf und Wasserstoff bei der üblichen Betriebstemperatur einer TLE-Anlage, also bei etwa 400 bis 550°C durch den Wärmetauscher zu leiten und dabei eine Behandlungsdauer von 1 bis 6 Stunden vorzusehen. Der Gehalt an Wasserstoff im Gemisch liegt zweckmäßigerweise bei 5 bis 20 Gewichts-%, vorzugsweise bei 5 bis 10 Gewichts-%. Der Druck des durchgeleiteten Gemisches kann dem üblichen Betriebsdruck einer TLE-Anlage entsprechen, also z.B. etwa 0,5 bis 20 bar betragen; ein bevorzugter Bereich liegt bei 1,5 - 2,5 bar.

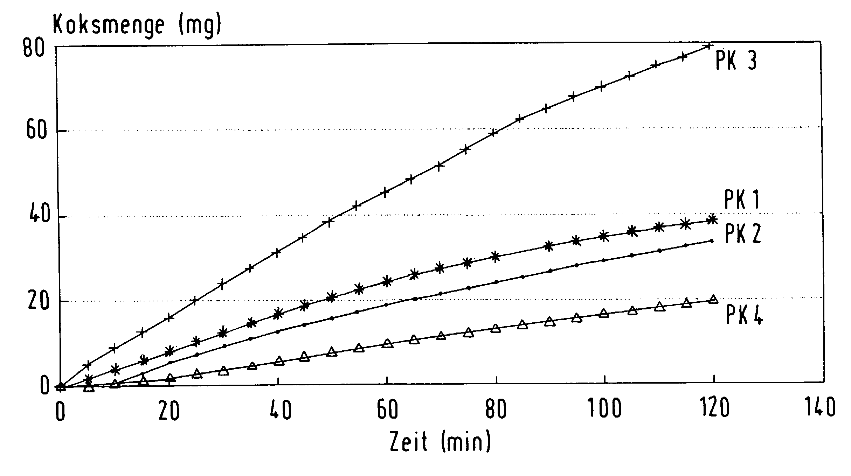

Nachfolgend wird die Erfindung anhand mehrerer Vergleichsbeispiele und erfindungsgemäßer ausführungsbeispiele näher erläutert. Die einzige Figur zeigt die Abhängigkeit der Koksbildung an einer Wärmetauschfläche von unterschiedlichen Nachbehandlungen nach einer Entkokung.

### Beispiel 1 (Vergleichsbeispiel)

In einer Laborpyrolyseapparatur stieg bei 500°C die Koksbildung an einem Probekörper innerhalb von 10 Versuchsminuten von einer Anfangsgeschwindigkeit (r_{c}(0)) von 15 µg/min (gemessen nach einer Versuchszeit von 5 min) auf eine maximale Koksbildungsgeschwindigkeit (r_{c}(max)) von 258 µg/min an. Im Laufe von weiteren 2 Std. stellte sich ein quasistationärer Wert von r_{c} (stat) = 65 µg/min ein. Nach der Entkokung mit Luft (5 l/h) stieg im nachfolgenden Pyrolysezyklus sowohl die maximale Koksbildungsrate r_{c} (max) als auch die quasistationäre Koksbildungsrate r_{c} (stat) stark an. In weiteren Pyrolyse-Entkokungszyklen setzte sich dieser Trend fort, wie aus Tabelle 1 zu entnehmen ist.

**Tabelle 1**

| Zyklus | r_{c} (0) [µg/min] | r_{c} (max) [µg/min] | r_{c} (stat) [µg/min] |
|---|---|---|---|
| 1. | 15 | 258 | 65 |
| 2. | 340 | 465 | 220 |
| 3. | 445 | 485 | 283 |
| 4. | 510 | 648 | 340 |
| 5. | 835 | 1043 | 530 |

### Beispiel 2 (erfindungsgemäßes ausführungsbeispiel)

In der gleichen Apparatur wie in Beispiel 1 wurde unter analogen äußeren Bedingungen technisches Propan pyrolysiert und die Koksbildung in der Postcrackingzone an 15Mo3-Probekörpern analoger Dimensionierung bei 500°C verfolgt.

Im Unterschied zu den vorstehenden Vergleichsbeispielen wurde nach Pyrolyse und Luftentkokung eine vierstündige Nachbehandlung des Probekörpers mit einem Gemisch aus Wasserstoff und Wasserdampf durchgeführt, wobei der Gesamtdurchsatz einheitlich 10 l/h betrug und das Volumen-Verhältnis H₂/H₂O von 9 - 0,1 geändert wurde.

In Tabelle 2 sind die Ergebnisse der Untersuchungen zusammengestellt.

**Tabelle 2**

| Vol-Verh. H₂/H₂O | r_{c} (0) [µg/min] | r_{c} (max) [µg/min] | r_{c} (stat) [µg/min] |
|---|---|---|---|
| ohne Nachbehandlg. | 340 | 465 | 200 |
| 9 | 1 150 | 1 150 | 500 |
| 4 | 470 | 470 | 180 |
| 2,5 | 110 | 410 | 180 |
| 1,5 | 35 | 310 | 170 |
| 0,7 | 0 | 345 | 160 |
| 0,1 | 0 | 500 | 520 |

Es ist ersichtlich, daß die erfindungsgemäße Nachbehandlung schon bei einem H₂/H₂O-Volumenverhältnis von 2,5 die Koksbildung in der Postcrackingzone zu Beginn des Pyrolyselaufs auf weniger als 1/3 gegenüber der Fahrweise ohne Nachbehandlung reduziert. Bei einer weiteren Absenkung der H₂-Anteile wird die Koksbildung zu Versuchsbeginn ganz unterdrückt; r_{c} (max) und r_{c} (stat) erreichen niedrigere Werte als ohne Nachbehandlung oder mit H₂-reichen H₂/H₂O-Gemischen. Bei sehr geringen Wasserstoffanteilen nimmt die Koksbildung wieder zu.

### Beispiel 3 (erfindungsgemäßes ausführungsbeispiel)

In der gleichen Apparatur wie in Beispiel 1 wurde unter analogen äußeren Bedingungen technisches Propan pyrolysiert und die Koksbildung in der Postcrackingzone an 15Mo3-Probekörpern analoger Dimensionierung bei 500°C verfolgt.

Dabei wurde die Nachbehandlung mit einem Gemisch aus 5 l/h Wasserstoff und 8,7 l/h Wasserdampf (Vol-Verh. H₂/H₂O = 0,6) ausgeführt und dabei die Behandlungsdauer zwischen 1 und 12 Stunden variiert. In Tabelle 3 sind die Koksbildungsraten der nachbehandelten Probekörper denjenigen gegenüber gestellt, die bei Pyrolyseversuchen ohne Nachbehandlung im 2. Pyrolysezyklus gemessen wurden.

Die Variation der Nachbehandlungsdauer zeigte, daß in allen Fällen zu Beginn der danach ausgeführten Pyrolyseversuche keine Koksbildung auftrat (r_{c} (0)=0), während ohne Nachbehandlung schon zu Beginn des Versuchs die Koksbildungsrate bei 340 µg/min lag. Die Tabelle 3 zeigt aber auch, daß bei sehr langer Nachbehandlung die Koksbildung wieder ansteigt.

**Tabelle 3**

| Nachbehandlung [h] | r_{c}(0) [µg/min] | r_{c}(max) [µg/min] | r_{c}(stat) [µg/min] |
|---|---|---|---|
| ohne | 340 | 465 | 200 |
| 1 | 0 | 370 | 155 |
| 6 | 0 | 195 | 140 |
| 12 | 0 | 295 | 160 |

### Beispiel 4 (Vergleich und Erfindung)

In einer Laborpyrolyseapparatur gemäß Beispiel 1 wurden vier Probekörper (PK 1-4) aus 15Mo3 in gleicher Weise unter Einsatz von technischem Propan verkokt. Im Anschluß daran wurden die einzelnen Probekörper jeweils unterschiedlichen Behandlungsprozeduren unterzogen und zwar:
- PK 1: Entkokung mit Luft (5 l/h)
- PK 2: Entkokung mit Luft (5 l/h) und Wasserdampf (6 l/h)
- PK 3: Entkokung mit Luft (5 l/h) und anschließend Behandlung mit Wasserdampf (17 l/h; 4 h)
- PK 4: Entkokung mit Luft (5 l/h) und anschließend Behandlung mit einem Gemisch aus Wasserstoff (5 l/h) und Wasser (7 l/h) bei einer Behandlungsdauer von 6h.

Somit wurde nur der Probekörper PK 4 erfindungsgemäß behandelt. Alle 4 Probekörper wurden erneut einer Verkokung mit technischem Propan unterzogen (Bedingungen wie in Beispiel 1) und die Koksbildung über einen Zeitraum von 2 h gemessen. Die Ergebnisse sind in der einzigen Figur graphisch wiedergegeben. Ein Vergleich zeigt, daß an dem erfindungsgemäß behandelten Probekörper PK 4 die bei weitem niedrigsten Koksbildungsraten gemessen wurden. Aus den ermittelten Gesamtkoksmengen (PK 1: m_{c} = 40,5 mg; PK 2: m_{c} = 38,2 mg; PK 3: m_{c} = 76,3 mg; PK 4: m_{c} = 24,1 mg;) ist zu schließen, daß die erfindungsgemäße Behandlung eine signifikante Laufzeitverlängerung gegenüber einer Fahrweise ohne H₂/H₂O-Behandlung ermöglicht (im gegebenen Beispiel ca. 40 %).

## Patentansprüche

1. Verfahren zur Verminderung der Verkokung von Wärmetauschflächen in einem Röhrenwärmetauscher aus einem Kesselbaustahl, der insbesondere innerhalb einer Anlage für das Thermocracken von Kohlenwasserstoffen zu Alkenen hinter dem Crackofen unter Erzeugung von Wasserdampf eine schnelle Abkühlung der Crackprodukte vornimmt,
dadurch gekennzeichnet,
daß die mit den Crackprodukten in Kontakt tretenden Seiten der Wärmetauschflächen nach einer durchgeführten Reinigung vor dem Wiedereinsatz des Röhrenwärmetauschers so lange unter reduzierenden Bedingungen behandelt werden, bis eine weitestgehende Reduktion von Fe₂O₃ an der Oberfläche zu Fe₃O₄ eingetreten ist, ohne daß dabei submikrones Fe-Pulver gebildet wird.

2. Verfahren nach Anspruch 1,
dadurch gekennzeichnet,
daß nach der Reinigung, insbesondere nach einer Reinigung mittels eines heißen Wasserdampf/Luft-Gemisches, eine Mischung aus heißem Wasserdampf und Wasserstoff auf der mit den Crackprodukten in Kontakt tretenden Seite durch den Röhrenwärmetauscher geleitet wird.

3. Verfahren nach Anspruch 2,
dadurch gekennzeichnet,
daß das Wasserdampf/Wasserstoff-Gemisch eine Temperatur im Bereich von 400 bis 550 °C aufweist.

4. Verfahren nach einem der Ansprüche 2 bis 3,
dadurch gekennzeichnet,
daß die Einwirkung des Wasserdampf/Wasserstoff-Gemisches auf die Wärmetauschflächen über eine Dauer von 1 bis 6 Stunden aufrechterhalten wird.

5. Verfahren nach einem der Ansprüche 2 bis 4,
dadurch gekennzeichnet,
daß der Gehalt an Wasserstoff im Wasserdampf/Wasserstoff-Gemisch 5 bis 20 Gewichts-%, insbesondere 5 bis 10 Gewichts-% beträgt.

6. Verfahren nach einem der Ansprüche 2 bis 5,
dadurch gekennzeichnet,
daß das Wasserdampf/Wasserstoff-Gemisch unter einem Druck von 0,5 bis 20 bar, insbesondere von 1,5 bis 2,5 bar durch den Röhrenwärmetauscher geleitet wird.

## Claims

1. A method for reducing the coking of heat-exchange surfaces in a tubular heat exchanger made of a boiler steel, which effects rapid cooling of the cracking products in particular within a plant for the thermal cracking of hydrocarbons to form alkenes after the cracking furnace, producing steam,
characterised in that the sides of the heat-exchange surfaces which come into contact with the cracking products, once cleaning has been effected, before reuse of the tubular heat-exchanger, are treated under reducing conditions until as extensive as possible a reduction of Fe₂O₃ on the surface to Fe₃O₄ has taken place, without submicron Fe powder being formed in so doing.

2. A method according to Claim 1, characterised in that after the cleaning, in particular after cleaning by means of a hot steam/air mixture, a mixture of hot steam and hydrogen is passed through the tubular heat-exchanger on the side which comes into contact with the cracking products.

3. A method according to Claim 2, characterised in that the steam/hydrogen mixture is at a temperature in the range from 400 to 550°C.

4. A method according to one of Claims 2 to 3, characterised in that the action of the steam/hydrogen mixture on the heat-exchange surfaces is maintained for a period of 1 to 6 hours.

5. A method according to one of Claims 2 to 4, characterised in that the content of hydrogen in the steam/hydrogen mixture is 5 to 20% by weight, in particular 5 to 10% by weight.

6. A method according to one of Claims 2 to 5, characterised in that the steam/hydrogen mixture is passed through the tubular heat-exchanger at a pressure of 0.5 to 20 bar, in particular of 1.5 to 2.5 bar.

## Revendications

1. Procédé permettant de réduire le cokage de surfaces d'échange de chaleur dans un échangeur de chaleur tubulaire, fait en un acier de construction pour la chaudronnerie, et qui exécute, notamment à l'intérieur d'une installation utilisée pour le craquage thermique d'hydrocarbures pour l'obtention d'alcènes, un refroidissement rapide des produits de craquage, après le four de craquage, en produisant de la vapeur d'eau,
caractérisé en ce que les côtés des surfaces d'échange de chaleur, qui viennent en contact avec les produits de craquage, sont traités, après l'exécution d'un nettoyage et avant la remise en service de l'échangeur de chaleur tubulaire, dans des conditions réductrices jusqu'à ce que se produise, à la surface, une réduction aussi étendue que possible du Fe₂O₃ en Fe₃O₄, sans qu'il y ait de formation de poussière de Fe d'ordre submicronique.

2. Procédé selon la revendication 1,
caractérisé en ce que, après le nettoyage, notamment après un nettoyage au moyen d'un mélange chaud de vapeur d'eau et d'air, un mélange de vapeur d'eau chaude et d'hydrogène est acheminé dans l'échangeur de chaleur tubulaire, sur le côté venant en contact avec les produits de craquage.

3. Procédé selon la revendication 2,
caractérisé en ce que le mélange de vapeur d'eau et d'hydrogène présente une température comprise dans la plage de 400 à 550 °C.

4. Procédé selon l'une des revendications 2 et 3,
caractérisé en ce que l'action du mélange de vapeur d'eau et d'hydrogène sur les surfaces d'échange de chaleur est maintenue pendant une durée allant de 1 à 6 heures.

5. Procédé selon l'une des revendications 2 à 4,
caractérisé en ce que la teneur en hydrogène du mélange de vapeur d'eau et d'hydrogène est comprise entre 5 et 20 % en poids et, notamment, entre 5 et 10 % en poids.

6. Procédé selon l'une des revendications 2 à 5,
caractérisé en ce que le mélange de vapeur d'eau et d'hydrogène est acheminé dans l'échangeur de chaleur tubulaire à une pression comprise entre 0,5 et 20 bars et, notamment comprise entre 1,5 et 2,5 bars.
